# EUROPEAN PATENT SPECIFICATION

(11) **EP 1 800 110 B1**
(45) Date of publication and mention of the grant of the patent: **19.03.2008**
(21) Application number: 04806871.2
(22) Date of filing: 17.12.2004
(51) Int. Cl.: G01N 21/35, G01N 21/01, B07C 5/342, G01N 33/02

(54) **DEVICE FOR INSPECTING VEGETABLE PRODUCTS BY SPECTROSCOPIC ANALYSIS OF REFRACTED LIGHT**
VORRICHTUNG ZUM INSPIZIEREN VON PFLANZLICHEN PRODUKTEN DURCH SPEKTRALANALYSE VON GEBROCHENEM LICHT
DISPOSITIF POUR CONTROLER DES PRODUITS VEGETAUX PAR ANALYSE SPECTROSCOPIQUE DE LUMIERE REFRACTEE

(30) Priority: 12.10.2004 IT RE20040129
(43) Date of publication of application: 27.06.2007
(73) Proprietor: SACMI COOPERATIVA MECCANICI IMOLA SOCIETA' COOPERATIVA, 40026 Imola (Bologna) (IT)
(72) Inventor: BELTRANDI, Dario, I-40026 Imola (IT)
(74) Representative: Corradini, Corrado
(86) International application number: PCT/IT2004/000706
(87) International publication number: WO 2006/040782

(56) References cited:
- EP-A- 1 279 950
- US-A- 4 884 696
- US-A1- 2003 156 281
- US-B1- 6 233 051
- US-B1- 6 435 002

## Description

### TECHNICAL FIELD

The present invention relates to quality control in general, with particular regard to the degree of ripening, of vegetable products in general and fruit in particular.

### PRIOR ART

Inspection devices are known comprising a conveyor for the fruit to be inspected, devices for illuminating the fruit located on the conveyor, and devices to receive the light transmitted through the inspected fruit and to transmit it to spectroscopic analysis means.

Devices are known, in particular from EP 1 215 480, for inspecting fruit to determine its quality, these comprising a conveyor chain, the links of which each consist of a cup-shaped seat on which the fruit to be inspected is placed; the cup is provided with a central hole through which a portion of the light refracted by the fruit passes, to be collected and concentrated by a suitable optical system and fed to a spectrometer for spectroscopic analysis.

For constructional and size reasons, the spectrometer cannot be located directly below the optical means, hence between these and the mm an optical fibre is located to conduct the light collected by the optical means to the spectrometer positioned at a distance.

The optical illumination system used in known systems comprises at least one series of lamps positioned on one side of the fruit, and causes the light rays to converge into a position within the fruit to be inspected.

The optical system for collecting the refracted light presents its focal point at a point within the fruit to be inspected which is different from the preceding.

The aforesaid inspection devices of known type present a series of limitations which prevent its large scale use.

A first serious drawback is that they require a conveyor for the fruit to be inspected which is very complicated and costly.

In this respect, each link of the conveyor chain must be constructed as a cup with its concavity facing upwards, on the edge of which the fruit rests; the cup must be holed at its centre.

The cup must also be of a particular design such that the fruit rests on the edge of the cup but without resting on its interior, this implying that the edge must be sufficiently wide to ensure stability of the fruit during conveying, but not sufficiently wide to enable the fruit to pass through. This means that each cup must be of dimensions able to receive fruit having its diameter within a fairly narrow range, implying that the same conveyor must be able to be fitted with sets of different sized cups, suitable for example for apricots, or for melons, to name two fruits of substantially different dimensions.

As each conveyor comprises from 200 to 250 cups, it is apparent that the different sets of cups which have to be available for the same conveyor represent a certain cost.

Plants are known for controlling and sorting fruit on the basis of certain characteristics, such as dimensions and colour, however the application of the present device to these already existing plants would involve an unreasonable cost, determined substantially by the cost involved in replacing all the cups of the conveyor.

Moreover, when the known inspection devices have been installed on one line, they cannot be easily shifted onto a different line; in other words the known inspection devices must be permanently associated with their respective transport line.

Furthermore, in US 6435002 a device is described comprising the features corresponding to the preamble of claim 1 of this invention.

### DISCLOSURE OF THE INVENTION

An object of the present invention is to provide an inspection device which can be associated with any transport line, provided it is able to transport fruits in equidistant positions, and which can be easily removed from one transport line and associated with another transport line.

A further object of the invention is to provide an inspection device which can be associated with existing sorting plants without requiring substantial and costly modifications thereto.

The objects of the invention are attained by a device having the characteristics listed in claim 1.

The other claims define characteristics which, when associated with those of claim 1, enable further advantages to be achieved.

### BRIEF DESCRIPTION OF THE DRAWINGS

The merits and characteristics of the invention will be more apparent from the ensuing detailed description given by way of non-limiting example and illustrated by the figures of the accompanying drawings.

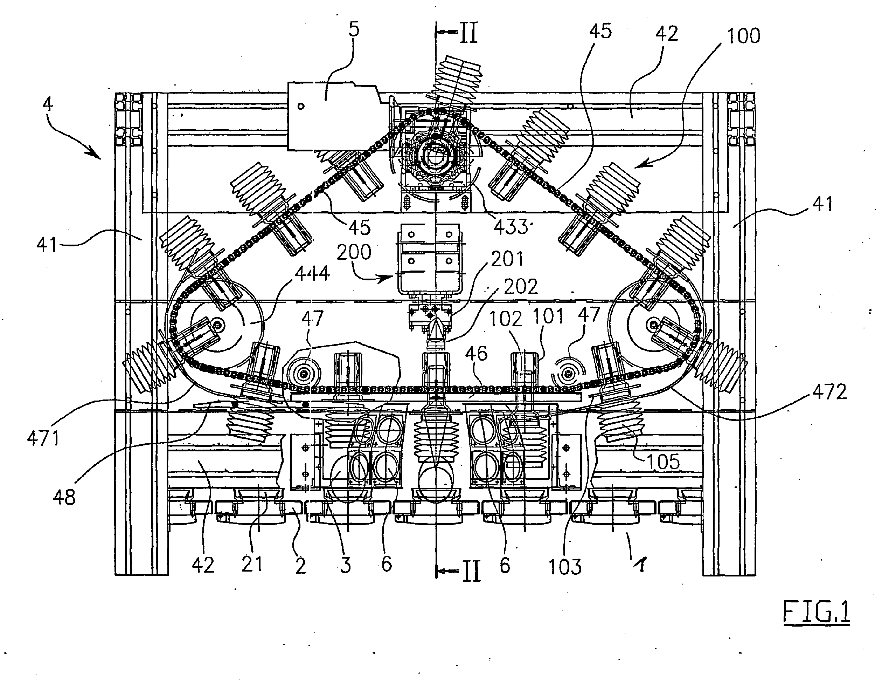

Figure 1 is a side view of the invention.
Figure 2 shows the section II-II indicated in Figure 1.

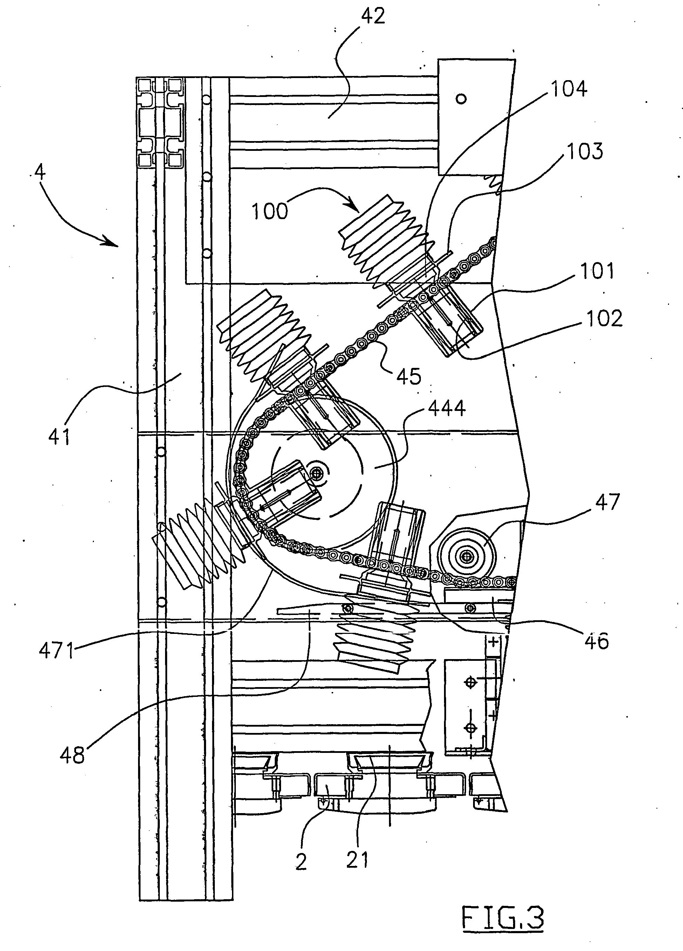

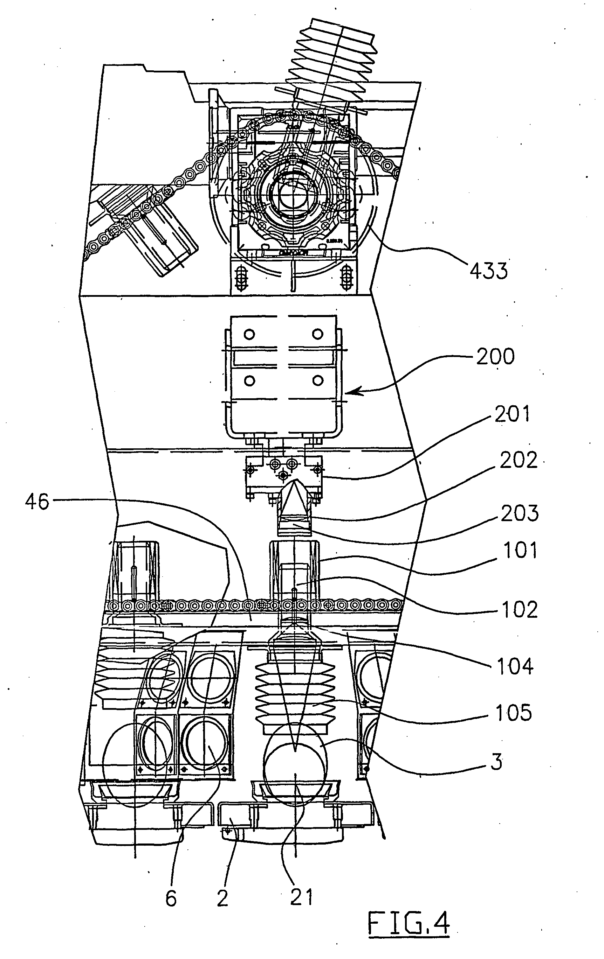

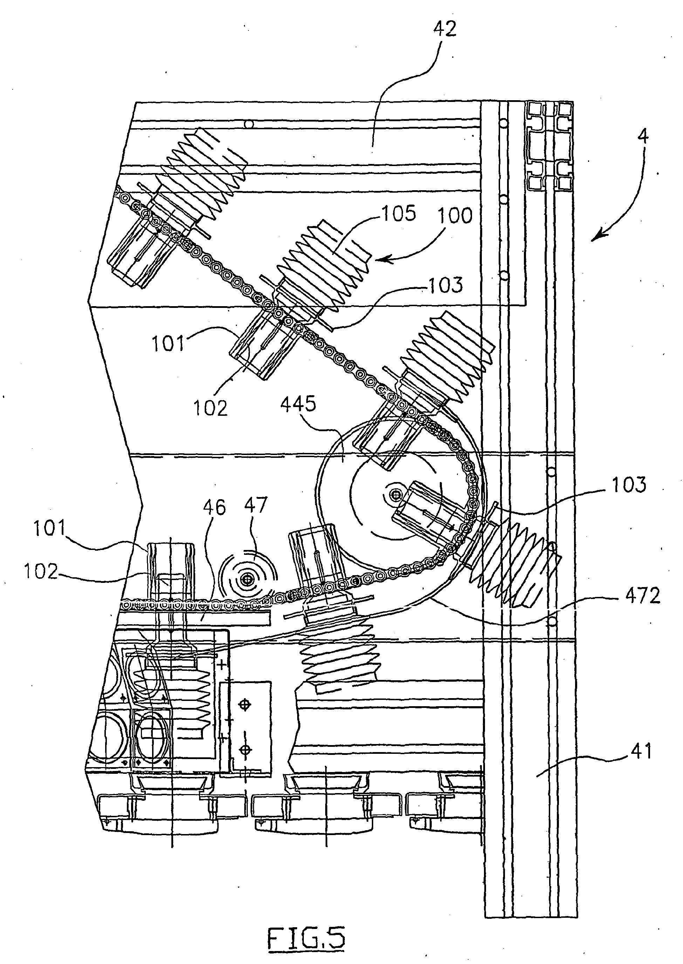

Figures 3, 4 and 5 show enlarged details of Figure 1.

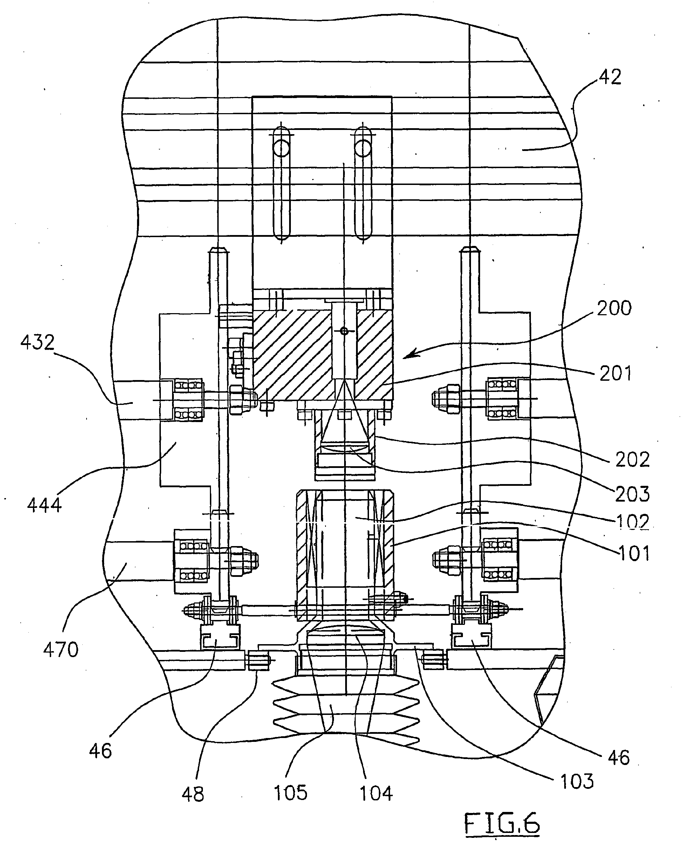

Figure 6 shows an enlarged part of Figure 2.

### BEST MODE FOR CARRYING OUT THE INVENTION

The figures show a conveyor 1 comprising a chain consisting of a series of equidistant links 2 for receiving a fruit 3 to be inspected.

Each link 2 comprises a seat 21 for receiving a fruit 3; the seat can be open lowerly or closed, as its characteristics have no influence on the invention. The essential condition is that it leaves free a lateral region of the fruit for its illumination.

The conveyor 1 is associated with an electric motor, not shown, for advancing it at constant, preferably adjustable, speed.

Along the lower straight portion of the conveyor, the chain links slide on a lower guide, not shown, which maintains them all perfectly aligned at the same height.

The inspection device of the present patent is positioned above the conveyor 1, and comprises a frame 4 positioned in correspondence with the straight portion of the conveyor 1.

In the illustrated example it comprises four uprights 41 connected together by suitable horizontal cross-members 42.

The frame presents three parallel horizontal axes 43 positioned at the vertices of a triangle having its horizontal base downwards.

The upper axis comprises a shaft 431 which is associated with a motorization unit 5 driven by an electric motor of controllable speed and advancement.

The lower axes comprise respectively two pairs of brackets 432 and 433 projecting from the sides of the frame 4. The shaft 43 carries keyed thereon a pair of sprocket wheels 443, while at the ends of the brackets of each pair 432, 433 there are positioned two coaxial idle sprocket wheels 444, 445, about which a pair of chains 45 extend, these also engaging the sprocket wheels 443.

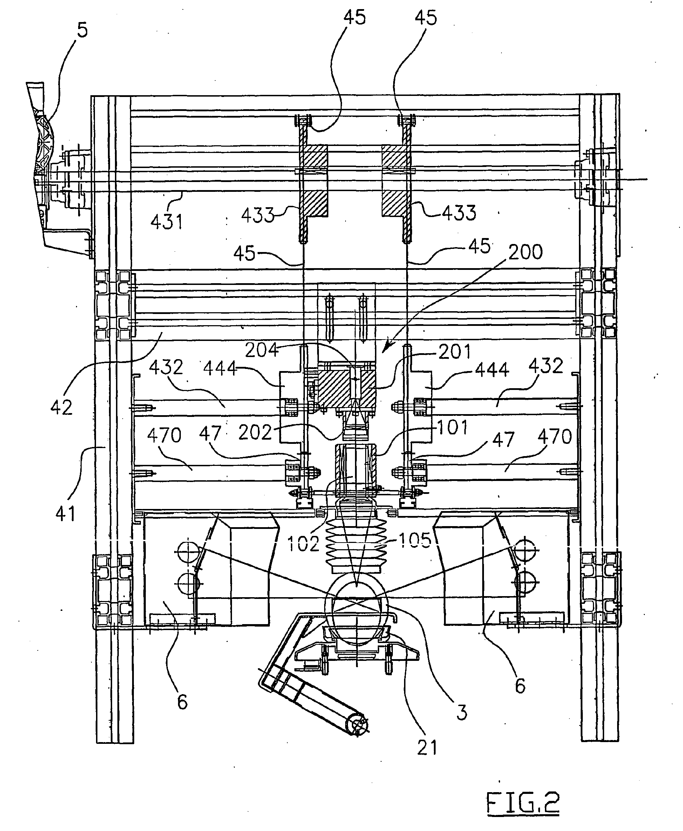

The central part of the lower portion of each chain 45 slides along a straight support guide 46 on which it is maintained by two pairs of coaxial sprocket wheels 47 positioned idle at the ends of two brackets 470 (Figure 2) supported by and extending from the sides of the frame 4.

Immediately below the two straight lower portions of the chains 45 there are positioned usual known illumination means 6 (Figure 2) which lie to the sides of the links of the conveyor 1, at the same level as the fruits positioned on them, in order to illuminate them in a direction transverse to the travel direction.

The actual inspection devices comprise a series of movable parts 100 which are fixed to the pair of chains 45 in equidistant positions and with the same pitch as the fruit reception seats 21 carried by the conveyor 1. Each of the movable parts 100 comprises a cylindrical body 101, fixed to the links of the chains 45, to telescopically receive a freely slidable inner cylindrical body 102 provided at the base of a projecting flange 103.

At the base of the body 102 there is positioned a hemispherical lens 104 able to project the incident light from below in the form of a beam of parallel rays directed upwards, with reference to the lower portions of the chains 45.

From the base of the body 102 there extends a cylindrical bellows 105 of soft material, such as rubber.

External to the sprocket wheels 444, 445 there are positioned guide and support means 471, 472 on which the flanges 103 of the bodies 102 rest, to prevent their withdrawal from the cylindrical bodies 101 by gravity.

As a continuation of guide means 471 positioned upstream in the travel direction of the chain 45, there is located a horizontal guide portion 48 on which the flanges 103 rest on abandoning the guide means 471; the guide means 472 positioned downstream in the travel direction of the chains 45 move downwards by a sufficient amount to receive the flanges 103 when the bodies 102 are lowered by gravity.

The sides of the frame 4 support the fixed part 200 of the inspection device in a central position with respect to the lower portion of the chains 45.

This part comprises an optical unit 201 for receiving the light refracted by the fruit transiting on the conveyor 1.

The optical unit 201 comprises a lower cylindrical body 202, of vertical axis, which aligns with the body 102 of the movable parts 100 of the device.

The body 202 is adjustable in level to the lle just above the top of the bodies 101, and comprises at its base a hemispherical lens 203 arranged to concentrate the parallel rays leaving the lens 203 positioned on the body 102; the end of an optical fibre 204 is positioned at the focal point of the lens 203, and leads to a remote spectrometer, not shown.

To the sides of the lower cylindrical body 202 there are positioned two angular plates 205 of sheet metal or other suitable material to prevent external light from reaching the lens 203 of the optical unit.

If the dimensions of the spectrometer allow it, it can be located directly above the optical unit.

A detailed description of the devices associated with the spectrometer for its operation, its calibration and the processing of data is omitted, as these are known and well described in the said document EP 1 215 480.

The device illustrated and described operates in the following manner. The inspection unit comprising the chains 45 is positioned above the conveyor 1, and the devices 100 are spaced apart at the same distance as that between the fruit receiving seats on the conveyor 1; the devices are then set into phase with the underlying seats 21 so that during the straight path of the chains 45 they lie perfectly coaxial with the seats 21. The speed of the conveyor 1 and of the chains 45 is then adjusted to the same value.

When the illumination devices 6 have been switched on, the conveyor 1 and the chains 45 are operated at the same speed.

When the movable parts 100 of the inspection device enter the straight portion, the flanges 103 of the lower telescopic bodies 102 abandon the guide 48 and descend by gravity until the lower end of the soft bellows 105 rests on the underlying fruit 3.

This is illuminated by the units 6, and when the body 101 transits immediately under the optical unit 201 of the fixed part 200, the light which has passed through the fruit reaches the lens 203 of the fixed part in the form of a beam of parallel rays which is concentrated onto the end of the optical fibre 204, through which it reaches the spectrometer.

The lateral protection plates 205 and the bellows 105 prevent the optical unit 201 from receiving any light other than that which has passed through the fruit, and which could disturb the measurements.

## Claims

1. A device for inspecting vegetable products in general, and fruit in particular, by spectroscopic analysis of refracted light, comprising a conveyor (1) provided with equidistant seats (21) for receiving single fruits (3), means (6) for laterally illuminating the fruits, and means (100, 200) arranged to collect the refracted light which passes through the fruit, to concentrate it and to feed it to spectroscopic analysis means, **characterised in that** the refracted light collection means comprise a fixed part (200) and a series of movable parts (100) positioned equidistant on a conveyor chain (45) lying above and parallel to a portion of the conveyor (1) and in phase with the seats (21) of this latter, said movable parts being formed from two telescopic bodies (101, 102), of which the outer body (101) is permanently fixed to the chain and the inner body (102) can slide freely downwards by gravity, the inner telescopic body 102 extending downwards as a soft bellows 105 open at the bottom.

2. A device as claimed in claim 1, **characterised in that** the conveyor chain (45) comprises a pair of identical side-by-side chains extending endlessly about deviation sprockets (433, 444, 445) in a triangular path with its flat base positioned downwards, with the sprockets (444, 445) positioned at the ends of the base there being associated guide means (471, 472) for preventing the inner telescopic body (102) withdrawing from the outer telescopic body (101) by gravity.

3. A device as claimed in claim 2, **characterised in that** the inner telescopic body (102) comprises a flat flange (103) arranged to interact with the guide means (471, 472).

4. A device as claimed in claim 2, **characterised in that** the guide means (471) positioned upstream in the travel direction of the chain (45) terminate above a horizontal flat guide (48).

5. A device as claimed in claim 2, **characterised in that** the guide means (472) positioned downstream in the travel direction of the chain (45) are arranged to receive the flange (103) when the inner telescopic body (102) has completely withdrawn.

6. A device as claimed in claim 1, **characterised in that** a hemispherical lens (104) is positioned at the base of the inner telescopic body (102) to collect the light originating from the fruit and to concentrate it into a beam of parallel rays directed upwards.

7. A device as claimed in claim 1, **characterised in that** the fixed part (200) of the refracted light collection means comprise an optical unit (201) provided with a cylindrical body (202) the axis of which is coplanar with the axes of the movable parts (100).

8. A device as claimed in claim 7, **characterised in that** the cylindrical body (202) comprises a hemispherical lens arranged to concentrate the beam of light rays originating from the movable part (100) onto the end of an optical fibre (204) leading to the remotely positioned spectroscopic analysis device.

9. A device as claimed in claim 8, **characterised in that** the cylindrical body (202) is adjustable in level such as to graze the top of the fixed telescopic body (101) of the fixed part (100).

10. A device as claimed in claim 9, **characterised in that** two downwardly facing angular plates (205) are positioned to the sides of the cylindrical body (202) to shield the light originating from the surrounding environment.

## Patentansprüche

1. Vorrichtung für die Untersuchung von pflanzlichen Produkten im Allgemeinen, insbesondere von Obst, mit Hilfe einer spektroskopischen Analyse mit gebrochenem Licht; sie besteht aus einem Förderer (1), der mit abstandsgleich angebrachten Aufnahmen (21) für Einzelfrüchte (3) ausgestattet ist, Beleuchtungskörpern (6) für die seitliche Beleuchtung der Früchte, sowie Geräten (100, 200), die so angeordnet sind, dass sie das durch die Frucht gehende gebrochene Licht sammeln, konzentrieren und an das spektroskopische Instrument weiterleiten.
Das Gerät, in dem das gebrochene Licht gesammelt wird, enthält ein starres Element (200) sowie eine Reihe von beweglichen Komponenten (100), die abstandsgleich auf einer über und parallel zu einem Abschnitt des Förderers (1) verlaufenden Luftkette (45) sowie in Übereinstimmung mit den Aufnahmen (21) angebracht sind; die beweglichen Komponenten bestehen aus zwei Teleskopschäften (101, 102), wobei der Außenschaft (101) permanent an der Kette befestigt ist, während sich der Innenschaft (102) der Schwerkraft folgend frei nach unten bewegen kann, und der ausziehbare Innenschaft (102) sich in Form eines weichen, an der Basis offenen Blasebalgs (105) nach unten ausdehnt.

2. Die im Patentanspruch 1 beschriebene Vorrichtung ist **dadurch** charakterisiert, dass die Luftkette (45) aus zwei identischen, nebeneinander liegenden Ketten besteht, die endlos auf Umlenkspulen (433, 444, 445) laufen und dabei ein mit der Basis nach unten liegendes Dreieck beschreiben. Die Spulen (444, 445) sitzen an den Endpunkten der Basis, wo die Führungselemente (471, 472) angeschlossen sind, um zu verhindern, dass sich der innere Teleskopschaft (102) aufgrund der Schwerkraft vom äußeren Teleskopschaft (101) ablöst.

3. Die im Patentanspruch 2 beschriebene Vorrichtung ist **dadurch** charakterisiert, dass am inneren Teleskopschaft (102) ein flacher Flansch (103) so angebracht ist, dass er mit den Führungselementen (471, 472) verbunden ist.

4. Die im Patentanspruch 2 beschriebene Vorrichtung ist **dadurch** charakterisiert, dass die oberhalb in Bewegungsrichtung der Kette (45) angebrachten Führungselemente (471) über einer horizontalen Führungsplatte (48) enden.

5. Die im Patentanspruch 2 beschriebene Vorrichtung ist **dadurch** charakterisiert, dass die unterhalb in Bewegungsrichtung der Kette (45) angebrachten Führungselemente (472) so liegen, dass sie den Flansch (103) aufnehmen, wenn der innere Teleskopschaft (102) vollständig eingezogen ist.

6. Die im Patentanspruch 1 beschriebene Vorrichtung ist **dadurch** charakterisiert, dass eine halbkugelförmige Linse (104) an der Basis des inneren Teleskopschafts (102) angebracht ist, um das von der Frucht ausgehende Licht zu sammeln und in einem Bündel aus nach oben gerichteten parallelen Strahlen zu konzentrieren.

7. Die im Patentanspruch 1 beschriebene Vorrichtung ist **dadurch** charakterisiert, dass das starre Element (200) des Gerätes, in dem das gebrochene Licht gebündelt wird, aus einer optischen Einheit (201) mit einem Rundkörper (202) besteht, dessen Achse koplanar zu den Achsen der beweglichen Elemente (100) verläuft.

8. Die im Patentanspruch 7 beschriebene Vorrichtung ist **dadurch** charakterisiert, dass der Rundkörper (202) eine halbkugelförmige Linse enthält, die so angebracht ist, dass das von der beweglichen Komponente (100) ausgehende Lichtstrahlenbündel an der Spitze der optischen Faser (204), die mit dem in entfernt aufgestellten spektroskopischen Instrument verbunden ist, konzentriert wird.

9. Die im Patentanspruch 8 beschriebene Vorrichtung ist **dadurch** charakterisiert, dass die Höhe des Rundkörper (202) einstellbar ist, sodass er die Oberkante des feststehenden Teleskopschafts (101) des starren Elements (100) berührt.

10. Die im Patentanspruch 9 beschriebene Vorrichtung ist **dadurch** charakterisiert, dass an den Seiten des Rundkörpers (202) zwei nach unten gerichtete Winkelplatten (205) angebracht sind, die das Umgebungslicht abschirmen.

## Revendications

1. Dispositif pour le contrôle de produits végétaux en général, et de fruits en particulier, au moyen d'une analyse spectroscopique à lumière réfractée, comprenant un convoyeur (1) équipé de logements (21) équidistants pour la réception de fruits (3) un par un, de moyens (6) pour l'éclairage latéral des fruits, et de moyens (100, 200) disposés de manière à recueillir la lumière réfractée qui passe à travers le fruit, la concentrant et la transmettant à l'instrument d'analyse spectroscopique,
**caractérisé par le fait que** le moyen qui permet de recueillir la lumière réfractée comprend un élément (200) fixe et une série de composants (100) mobiles positionnés de manière équidistante sur une chaîne (45) aérienne située au-dessus et en position parallèle à un tronçon du convoyeur (1) et en phase avec les logements (21) de ce dernier, tandis que les composants mobiles susmentionnés sont formés de deux corps (101, 102) télescopiques, dont le corps (101) externe est fixé de manière permanente à la chaîne, tandis que le corps (102) interne peut glisser librement vers le bas par gravité, et le corps 102 télescopique interne s'étend vers le bas sous forme d'un soufflet 105 souple ouvert à sa base.

2. Dispositif conforme à la revendication 1, **caractérisé par le fait que** la chaîne (45) aérienne comprend une paire de chaînes identiques positionnées une à côté de l'autre qui s'étendent sans fin sur des pignons (433, 444, 445) de déviation selon un parcours triangulaire dont la base plate est positionnée vers le bas, avec les pignons (444, 445) situés aux extrémités de la base où sont associés des éléments (471, 472) de guidage pour éviter que le corps (102) télescopique interne se décroche du corps (101) télescopique externe par gravité.

3. Dispositif conforme à la revendication 2, **caractérisé par le fait que** le corps (102) télescopique interne comprend une bride (103) plate positionnée de façon à interagir avec les éléments (471, 472) de guidage.

4. Dispositif conforme à la revendication 2, **caractérisé par le fait que** les éléments (471) de guidage positionnés en amont dans la direction de translation de la chaîne (45) finissent au-dessus d'une glissière (48) plate horizontale.

5. Dispositif conforme à la revendication 2, **caractérisé par le fait que** les éléments (472) de guidage positionnés en aval dans la direction de translation de la chaîne (45) sont situés de manière à recevoir la bride (103) si le corps (102) télescopique interne est complètement retiré.

6. Dispositif conforme à la revendication 1, **caractérisé par le fait qu'**une lentille (104) hémisphérique est positionnée à la base du corps (102) télescopique interne pour recueillir la lumière prenant son origine du fruit et la concentrer en un faisceau de rayons parallèles dirigés vers le haut.

7. Dispositif conforme à la revendication 1, **caractérisé par le fait que** l'élément (200) fixe de l'instrument de récupération de la lumière réfractée comprend une unité (201) optique équipée d'un corps (202) cylindrique, dont l'axe est coplanaire aux axes des composants (100) mobiles.

8. Dispositif conforme à la revendication 7, **caractérisé par le fait que** le corps (202) cylindrique comprend une lentille hémisphérique positionnée de manière à concentrer le faisceau de rayons prenant leur origine du composant (100) mobile sur l'extrémité de la fibre (204) optique reliée au dispositif d'analyse spectroscopique positionné à distance.

9. Dispositif conforme à la revendication 8, **caractérisé par le fait que** le corps (202) cylindrique est positionné à un niveau réglable, de manière à résulter rasant par rapport à la partie supérieure du corps (101) télescopique fixe de l'élément (100) fixe.

10. Dispositif conforme à la revendication 9, **caractérisé par le fait que** deux plaques (205) angulaires dirigées vers le bas sont positionnées sur les côtés du corps (202) cylindrique pour masquer la lumière prenant origine du milieu environnant.
